# FASCICULE DE BREVET EUROPEEN

(11) **EP 4 440 515 B1**
(45) Date de publication et mention de la délivrance du brevet: **14.01.2026**
(21) Numéro de dépôt: 22826152.5
(22) Date de dépôt: 23.11.2022
(51) Int. Cl.: A61F 9/00, A61J 1/14, B65D 47/18

(54) **FLACON POUR LA DISTRIBUTION GOUTTE À GOUTTE D'UN PRODUIT LIQUIDE STÉRILE CONTENANT UN AGENT TENSIOACTIF**
SPENDER ZUR TROPFAUSGABE EINES STERILEN FLÜSSIGEN PRODUKTS MIT EINEM TENSID
DISPENSER FOR DRIP-DISPENSING A STERILE LIQUID PRODUCT CONTAINING A SURFACTANT

(30) Priorité: 29.11.2021 FR 2112639
(43) Date de publication de la demande: 09.10.2024
(73) Titulaire: Laboratoires THEA, 63100 Clermont-Ferrand (FR)
(72) Inventeur: QUAGLIA, Benjamin, 63119 Châteaugay (FR)
(74) Mandataire: Santarelli
(86) Numéro de dépôt international: PCT/FR2022/052164
(87) Numéro de publication internationale: WO 2023/094769

(56) Documents cités:
- EP-A1- 1 593 605
- EP-A1- 1 683 737
- WO-A1-02/38464
- US-B2- 6 708 850

## Description

La présente invention porte sur un flacon pour la distribution goutte à goutte d'un produit liquide stérile contenant un agent tensioactif. Le flacon proposé selon la présente invention présente un intérêt tout particulier pour le conditionnement d'un produit ophtalmique liquide sans conservateur, contenant un agent tensioactif.

Il existe des produits liquides devant être distribués goutte à goutte dans de nombreux domaines de l'industrie, en particulier dans le domaine pharmaceutique, le domaine de la cosmétique, l'industrie alimentaire, etc. Ces produits sont utilisés par petites doses, d'une ou quelques gouttes issues d'un flacon lors de chaque utilisation, alors que l'on doit conserver le reste du produit contenu dans le flacon pendant un certain temps, généralement plusieurs jours voire plusieurs semaines. Le produit contenu dans le flacon doit généralement être maintenu à l'abri de toute contamination par des agents bactériens ou autres provenant de l'extérieur du flacon.

C'est en particulier le cas pour les compositions pharmaceutiques, parmi lesquelles en particulier les solutions ophtalmiques, qui constituent l'application préférentielle de la présente invention. Il existe en outre un intérêt particulier de proposer des solutions ophtalmiques sans conservateur. En effet, les produits utilisés en tant que conservateur dans les solutions ophtalmiques, pour assurer la stérilité des produits ophtalmiques vis-à-vis des bactéries et des champignons, peuvent avoir des effets indésirables importants.

Les conservateurs, notamment le chlorure de benzalkonium, sont irritants pour les yeux et peuvent altérer le film lacrymal, et peuvent provoquer une atteinte tissulaire par apoptose. L'utilisation à long terme de conservateurs a des conséquences indésirables sur l'oeil.

Ainsi, l'utilisation de produits ophtalmiques sans conservateurs est bien souvent recommandable.

Une première solution pour conserver la stérilité de tels produits ophtalmiques sans conservateurs consiste à les conditionner en doses uniques, aussi appelées « unidoses ». Le conditionnement en unidose n'est cependant pas applicable à tous les produits, et génère de nombreux déchets, le conditionnement de chaque dose devant être jeté après l'utilisation. Pour un traitement de longue durée, le conditionnement en unidose n'apparait ainsi pas approprié.

Dans ce contexte, des flacons contenant plusieurs doses, ou flacon « multidoses », configurés pour assurer la stérilité du produit qu'ils contiennent, y compris après ouverture et délivrance d'une première dose, ont été développés. Ces flacons ont pour but de conserver la stérilité dans le flacon tant que dure la consommation du produit jusqu'à l'épuisement complet du contenu du flacon.

Par exemple, le document FR2816600 divulgue un flacon de type goutte à goutte, dont le réservoir comporte une paroi déformable permettant de créer une surpression entrainant le passage du produit contenu au travers d'une membrane stérilisante. La surface de cette membrane est partiellement hydrophile et partiellement hydrophobe, et plus particulièrement sélectivement perméable aux liquides aqueux en sa partie hydrophile et sélectivement perméable à l'air en sa partie hydrophobe. Ainsi, le liquide traverse la membrane en sa partie hydrophile lors de sa distribution avant d'atteindre un embout de distribution goutte à goutte, tandis que de l'air peut être aspiré dans le flacon au travers de la partie hydrophobe pour compenser le volume de liquide distribué lorsque le réservoir n'est plus comprimé et revient à sa forme initiale. Un tampon poreux permet une régulation du flux dans le flacon, nécessaire à la bonne formation des gouttes.

Ce type de flacon est pleinement satisfaisant pour conditionner et distribuer de nombreux produits stériles sans conservateurs, et notamment des produits ophtalmiques liquides sans conservateur.

Néanmoins, ils ne sont pas adaptés à la distribution de certains produits. En particulier, les produits ophtalmiques contenant un agent tensioactif ne peuvent pas être distribués de façon satisfaisante par ce type de flacon. En effet, les agents tensioactifs interagissent avec la partie hydrophobe de la membrane (celle-ci étant obtenue par un traitement physico-chimique localisé de la membrane hydrophile) et la rendent hydrophile. Cela empêche le retour d'air dans le flacon, nécessaire pour compenser la quantité de liquide distribuée. Cela a pour conséquence une augmentation de la dépression dans le flacon au fur et à mesure de son utilisation, ce qui peut entrainer une déformation résiduelle du flacon et/ou une rupture de la membrane.

Un tensioactif (ou agent tensioactif ou agent de surface), est un composé qui modifie la tension superficielle entre deux surfaces. Ce sont des composés qui présentent deux parties de polarité différente, respectivement lipophile et hydrophile. Ils permettent de solubiliser deux phases non miscibles. Dans le cadre des produits ophtalmiques, les agents tensioactifs sont ainsi utilisés comme agent de solubilisation. Les termes agent tensioactif et agent de solubilisation sont ainsi équivalents dans le présent document. Le document FR2955842 divulgue un flacon amélioré réduisant la formation de mousse ainsi que la rétention d'air dans le tampon poreux. Néanmoins, si le flacon proposé améliore sensiblement la qualité de la distribution d'un produit contenant, soit un principe actif ayant lui-même des propriétés tensio-actives, soit des additifs tensio-actifs utilisés comme agents solubilisants, soit d'autres excipients, comme certains agents viscosants ou lubrifiants de la famille des dérivés polyvinyliques ou de celle des polyéthylène-glycols, il ne règle pas la problématique de l'incompatibilité de certains agents tensioactifs vis-à-vis de la partie hydrophobe de la membrane utilisée.

Le document FR2770495 divulgue un flacon pour produit ophtalmique liquide sans conservateur qui serait adapté à la distribution d'un produit contenant un agent tensioactif. Néanmoins, le flacon proposé est fondé sur un réservoir à paroi flexible, déformable manuellement en diminution progressive de son volume interne vers un col rigide, c'est-à-dire en pratique sur un réservoir déformable en accordéon, dont la manipulation n'est pas parfaitement aisée pour l'utilisateur.

Le document EP1985543 présente un flacon pour produit ophtalmique. Le réservoir du flacon est du type « à délaminage », ce qui signifie que la couche interne du réservoir peut se séparer du reste des parois pour former une poche interne déformable. Ce flacon comporte une valve anti-retour traversée par le produit sortant du flacon et peut comporter, en aval de cette valve, un filtre permettant le filtrage de certaines bactéries. Ce flacon nécessite cependant l'emploi d'une valve anti-retour très efficace, et a une configuration complexe lorsqu'une fonction de filtration est mise en œuvre.

Le document EP1683737 divulgue un flacon de type goutte à goutte équipé d'un filtre, et dont la configuration vise à empêcher la formation de bulles lorsqu'un liquide contenu doit être délivré à travers un orifice de délivrance du flacon.

L'invention vise à proposer un flacon pour produit liquide stérile contenant au moins un agent tensioactif qui résout au moins certains des problèmes mentionnés ci-dessus. Ainsi, l'invention porte sur un flacon pour la distribution goutte à goutte d'un produit liquide stérile contenant un agent tensioactif, comportant un réservoir comportant une paroi définissant un volume interne et adaptée à se déformer sous l'effet d'une pression exercée sur le réservoir par un utilisateur du flacon et à reprendre spontanément sa forme d'origine après relâchement de ladite pression. Le réservoir comporte en outre une poche souple dans le volume interne défini par la paroi et adaptée à contenir le produit liquide stérile. Le flacon comporte un moyen d'entrée d'air entre la paroi du réservoir et la poche souple, et une tête rapportée sur un col du réservoir et comportant un embout goutte à goutte comportant un orifice de distribution. Le flacon comporte en outre une membrane microporeuse stérilisante hydrophile, disposée de sorte à être traversée par le liquide issu de la poche souple en vue de la délivrance dudit liquide par l'embout goutte à goutte.

Le flacon comporte un dispositif configuré pour maintenir la membrane mouillée par du produit liquide issu du réservoir entre deux distributions de produit liquide. La membrane microporeuse est sélectivement perméable au liquide, c'est-à-dire qu'elle permet le passage d'un liquide aqueux tandis qu'elle s'oppose au passage des gaz lorsqu'elle est mouillée, de sorte que la membrane s'oppose au retour d'air dans la poche souple du réservoir lorsque le flacon reprend sa forme d'origine après une distribution du produit liquide stérile, le volume de liquide distribué étant compensé par l'entrée d'un volume d'air correspondant entre la paroi du réservoir et la poche souple via le moyen d'entrée d'air.

Le dispositif configuré pour maintenir la membrane mouillée entre deux instillations comporte un clapet positionné entre la membrane et l'orifice de distribution de l'embout goutte à goutte.

La présente invention propose ainsi un flacon de distribution goutte à goutte parfaitement adapté aux produits contenant un agent de solubilisation (tensioactif). Le flacon proposé est d'utilisation simple pour l'utilisateur, en ce qu'il permet la distribution de produit par simple pression sur le réservoir, ce qui est intuitif. En outre, la poche souple qui est présente dans le volume interne défini par la paroi du réservoir permet une distribution complète du produit qu'elle contient, c'est-à-dire que toutes les doses de produit contenues dans la poche souple peuvent être effectivement distribuées. La distribution de produit peut être également réalisée tandis que le flacon est positionné selon diverses orientations. En effet, au fur et à mesure que des doses de produit sont distribuées, le volume de produit distribué est compensé par l'entrée d'un volume équivalent d'air entre la paroi déformable du réservoir et la poche souple. Le volume de la poche souple s'adapte à celui du produit qu'elle contient. Le produit contenu dans la poche souple demeure ainsi à proximité immédiate de la tête de distribution, et aucune bulle d'air ne se forme dans la poche souple. Lorsque l'utilisateur applique une pression sur la paroi déformable, l'air présent entre la paroi déformable du réservoir et la poche souple est empêché de sortir de cet espace (que ce soit par un moyen qui empêche automatiquement cette sortie de l'air ou par une action de l'utilisateur), de sorte que la poche souple est elle-même mise en pression, provoquant l'expulsion du produit au travers de la membrane hydrophile et de l'embout goutte à goutte.

La membrane stérilisante garantit la stérilité du produit lors de sa délivrance. Elle protège également le produit contenu dans le réservoir contre toute contamination provenant de l'extérieur du flacon. Le flacon proposé dans l'invention tire en outre profit du caractère sélectivement perméable à l'eau (ou plus généralement aux liquides aqueux) de la membrane stérilisante pour éviter le retour d'air dans la poche souple après distribution du produit. En d'autres termes, la membrane, dont la capacité de filtration garantit la stérilité du produit contenu dans le flacon, ne permet pas le retour d'air dans le flacon et sert ainsi de dispositif anti-retour pour le flacon.

Afin de garantir l'absence totale de passage d'air, la membrane doit être mouillée. Cela est nécessairement le cas immédiatement après la distribution du produit, de sorte que la membrane joue correctement son rôle de dispositif anti-retour et empêche l'aspiration d'air dans la poche souple en compensation du volume de produit liquide distribué. Néanmoins, pour assurer que la membrane reste mouillée entre deux distributions de produit, qui sont généralement espacées de plusieurs heures si le produit est un produit ophtalmique, il est proposé de prévoir un dispositif adapté à garantir que la membrane reste mouillée. Ce dispositif peut être constitué de diverses manières. Une réserve de liquide peut être formée en amont de la membrane, ou une très faible quantité de liquide peut être maintenue en aval de la membrane.

Dans l'ensemble du présent document les termes « amont » et « aval » s'entendent en considérant le sens d'écoulement du produit liquide lors de sa délivrance, c'est-à-dire depuis le réservoir jusqu'à sa sortie de l'embout du flacon.

Il est notable que le clapet du dispositif configuré pour maintenir la membrane mouillée ne vise pas à assurer la fonction anti-réaspiration d'air dans le réservoir, qui est garantie dans l'invention par la membrane hydrophile, sélectivement perméable à l'eau. Un tel clapet assure simplement le maintien du produit liquide mouillant la membrane au niveau de celle-ci, en particulier entre deux distributions successives de produits. Un clapet simple, peu coûteux, peut ainsi être utilisé. Le clapet peut être un clapet à bille, un clapet à lamelles déformables, etc.

Le flacon proposé dans l'invention peut présenter une configuration générale simple. En particulier, il n'est pas nécessaire de prévoir un dispositif anti-retour parfaitement étanche à l'air en plus de la membrane pour empêcher le retour d'air dans la poche souple du réservoir lorsque la paroi reprend sa forme d'origine après avoir été déformée par pression pour entrainer la distribution de quelques gouttes de produit.

La poche souple peut être formée par délamination d'une couche interne du réservoir. En alternative à une poche souple présente dès la fabrication dans le réservoir du flacon, une poche souple obtenue par délamination de la paroi du réservoir est une solution simple, maitrisée industriellement, et peu coûteuse, pour obtenir la fonction recherchée dans l'invention.

Alternativement, la poche souple peut être une poche souple rétractable, qui est rapportée. L'emploi d'une telle poche souple rétractable permet notamment de sélectionner une poche ayant une contenance nominale correspondant précisément au volume initial de produit liquide à conditionner, tout en employant une même paroi déformable pour différents volumes de produit à conditionner.

Le moyen d'entrée d'air peut être formé d'un trou dans la paroi du réservoir adapté à être obturé par un doigt de l'utilisateur.

Il s'agit d'une solution particulièrement simple pour empêcher la sortie de l'air contenu entre la paroi du réservoir et la poche souple lors de la distribution de produit vers l'extérieur du réservoir, c'est-à-dire lorsqu'une pression est appliquée sur le réservoir. Un positionnement adapté du trou ménagé dans la paroi rend l'utilisation du flacon parfaitement intuitive. Le trou peut ainsi être avantageusement positionné en une zone du réservoir où un doigt de l'utilisateur vient naturellement se poser lorsqu'il tient le flacon en vue de distribuer du produit contenu par le flacon. Le trou peut ainsi être positionné sur une paroi latérale du réservoir, notamment à proximité du col du réservoir, ou, alternativement, sur le fond du réservoir.

Alternativement, le moyen d'entrée d'air peut comporter un clapet unidirectionnel, configuré pour permettre l'entrée d'air entre la paroi du réservoir et la poche souple et pour empêcher la sortie vers l'extérieur du réservoir de l'air présent entre la paroi du réservoir et la poche souple.

Selon cette configuration, la sortie d'air vers l'extérieur du réservoir est empêchée automatiquement, ce qui rend l'utilisation du réservoir extrêmement simple, car l'utilisateur n'a aucune action particulière à faire (aussi simple soit-elle) comparativement à un flacon classique à réservoir déformable.

Le dispositif configuré pour maintenir la membrane mouillée entre deux instillations peut comporter un capuchon amovible, rapporté sur la tête de distribution.

Un capuchon constitue une manière simple et efficace de fournir une étanchéité en aval (côté embout goutte à goutte) de la membrane. Une telle étanchéité évite le retour du liquide qui mouille la membrane vers la poche souple entre deux instillations.

Le flacon peut comporter un tampon poreux situé entre le réservoir et la membrane microporeuse et disposé de sorte à être traversé par le liquide issu de la poche souple en vue de la délivrance dudit liquide par l'embout goutte à goutte.

Le tampon poreux en amont de la membrane permet d'éviter que le produit liquide ne vienne au contact de la membrane avant la première distribution de produit. Avant la première utilisation, le liquide reste ainsi parfaitement confiné dans le réservoir, et aucune goute de liquide ne risque de traverser la membrane et de se retrouver ainsi dans une zone, au niveau de l'embout, où il est plus difficile de garantir sa stérilité.

Le tampon poreux permet également de créer une perte de charge sur le trajet du produit vers l'orifice de distribution, conjointement avec la membrane microporeuse, qui permet de réguler le flux de liquide distribué par le flacon. En d'autres termes, le tampon permet à l'utilisateur de doser plus facilement le nombre de gouttes souhaitées, en régulant le flux de liquide en sortie du flacon.

Si le tampon poreux est positionné au contact de la membrane, il peut également constituer un dispositif permettant de maintenir la membrane mouillée par du produit liquide issu du réservoir entre deux distributions de produit liquide. Cette disposition doit néanmoins être complétée d'un moyen évitant l'évaporation du produit liquide, par exemple un capuchon amovible.

Un tampon microporeux donnant satisfaction est avantageusement réalisé en une matière inerte vis-à-vis du liquide contenu dans le flacon. Des matières appropriées seront en particulier les feutres ou les mousses à haute porosité en pores ouverts telles qu'on sait les obtenir à partir de résines diverses de polymères organiques. Dans les principales applications de l'invention, il est avantageux de réaliser le tampon microporeux sous la forme d'une pastille de feutre de résines de polyesters ou polyesters modifiés, telles en particulier que les résines de polyéthylène à basse densité ou les résines de polyéthersulfone.

Les résines de ce type ou équivalentes ont l'intérêt, dans le cadre de l'invention, de se prêter à fournir un tampon cylindrique, dans les diamètres de 0,5 à 3 cm et des longueurs comprises entre 0,2 et 1 cm, qui présente une souplesse suffisante pour s'adapter en force de manière étanche dans le corps, avantageusement cylindrique, de la tête de distribution, et qui, dans le sens longitudinal, offre au passage du liquide des microcanaux d'un diamètre moyen de pores pouvant être choisis entre 0,3 et 10 microns.

La membrane microporeuse peut par exemple avoir une taille de pores inférieure à 0,45 µm et de préférence inférieure ou égale à 0,22 µm.

Une telle membrane offre une filtration dite stérilisante en ce qu'elle empêche le passage des bactéries, des champignons, et, selon la porosité choisie, de certains virus. La membrane peut être constituée de divers matériaux, par exemple du type PES, nylon, PVDF. Elle évite le passage d'éventuels contaminants lors de la distribution du produit (bien que normalement le produit soit conditionné de manière stérile, de sorte qu'aucun contaminant n'y est normalement présent), mais garantit aussi la stérilité du produit contenu dans le réservoir en empêchant le passage dans le réservoir de contaminants (bactéries, voire virus) depuis l'extérieur du flacon. Aucun conservateur n'est ainsi nécessaire dans le produit.

Les surfaces du flacon situées entre la membrane microporeuse et l'orifice de distribution peuvent porter un revêtement bactéricide, par exemple à base d'ions argent ou d'ion zinc. La membrane microporeuse peut comporter un agent bactéricide, par exemple à base d'ions argent ou d'ion zinc.

Le traitement des surfaces du flacon qui sont en aval de la membrane et qui sont susceptibles d'être au contact du produit lors de la distribution, voire au contact de produit restant dans l'embout goutte à goutte suite à une distribution permet de garantir la stérilité du produit jusqu'à sa sortie du flacon. Un tel traitement évite de manière générale toute prolifération bactérienne sur les surfaces mouillées du flacon. Le traitement de ces surfaces peut consister en l'application d'un revêtement sur ces surfaces. Alternativement, le matériau constituant ces surfaces peut être chargé dans sa masse d'un agent bactéricide. Les agents bactéricides à base d'ions métalliques, par exemple les ions argent ou zinc sont préférés pour les applications de l'invention au conditionnement d'un produit ophtalmique.

La membrane microporeuse est ainsi avantageusement située au plus près de l'orifice de distribution du flacon.

Le flacon peut comporter dans son réservoir un produit ophtalmique liquide stérile sans conservateur et contenant au moins un agent tensioactif.

Il s'agit de l'application préférentielle de l'invention. L'invention permet un conditionnement d'un tel produit sous forme multidoses, dans un flacon simple, peu coûteux, et pratique pour l'utilisateur.

Le produit ophtalmique liquide stérile peut par exemple comporter entre 0,1% et 5% en volume d'agent tensioactif.

L'au moins un agent tensioactif peut comporter un ou plusieurs des agents choisis parmi :
Polyéthylène glycol 400 (PEG 400) ;
Monooléate de polyoxyéthylène sorbitane (polysorbate 80) ;
Monolaurate de polyoxyéthylène sorbitane (polysorbate 20) ;
Oxyde d'éthylène, oxyde de propylène ;
Polymère de méthyloxirane ;
Huile de ricin PEG-35 ;
Huile de ricin hydrogénée PEG-40 ;
éther cétostéarylique de macrogol ;
Polyoxypropyle glycol 407 ;
Vitamine E (polyéthylène glycol succinate) ;
Hydroxystéarate de macrogol 15 ;
Caprylocaproyl macrogol-8 glycérides ;
2-(2-éthoxyéthoxy) éthanol ;
Polyvinylpyrrolidone.

Le produit ophtalmique liquide peut comporter au moins un additif choisi dans le groupe des agents isotonisants de type non ionique, des agents anti-oxydants , des systèmes tampons, des polymères lubrifiants, des oligosaccharides. Le produit ophtalmique peut en outre comporter un ou plusieurs actifs en quantité thérapeutiquement efficace choisi dans le groupe des anti-glaucomateux, des anti-inflammatoires, des immuno-supresseurs, des antiviraux, des antibactériens ou des antifongiques.

D'autres particularités et avantages de l'invention apparaîtront encore dans la description ci-après.

Aux dessins annexés, donnés à titre d'exemples non limitatifs :
- la figure 1 représente, selon une vue en coupe, un flacon conforme à un mode de réalisation de l'invention ;
- la figure 2 représente, selon une vue en coupe, le flacon de la figure 1 lors de la délivrance du produit qu'il contient ;
- la figure 3 représente, selon une vue en coupe, un flacon conforme à un mode de réalisation de l'invention, illustrant plusieurs aspects pouvant être mis en oeuvre dans un tel flacon ;
- la figure 4 représente, selon une vue en coupe, un flacon conforme à un autre mode de réalisation de l'invention, illustrant plusieurs aspects pouvant être mis en oeuvre dans un tel flacon ;
- la figure 5 représente, selon une vue en coupe, un flacon conforme à un autre mode de réalisation de l'invention ;
- la figure 6 représente, selon une vue en coupe partielle, un flacon conforme à encore un autre mode de réalisation de l'invention.

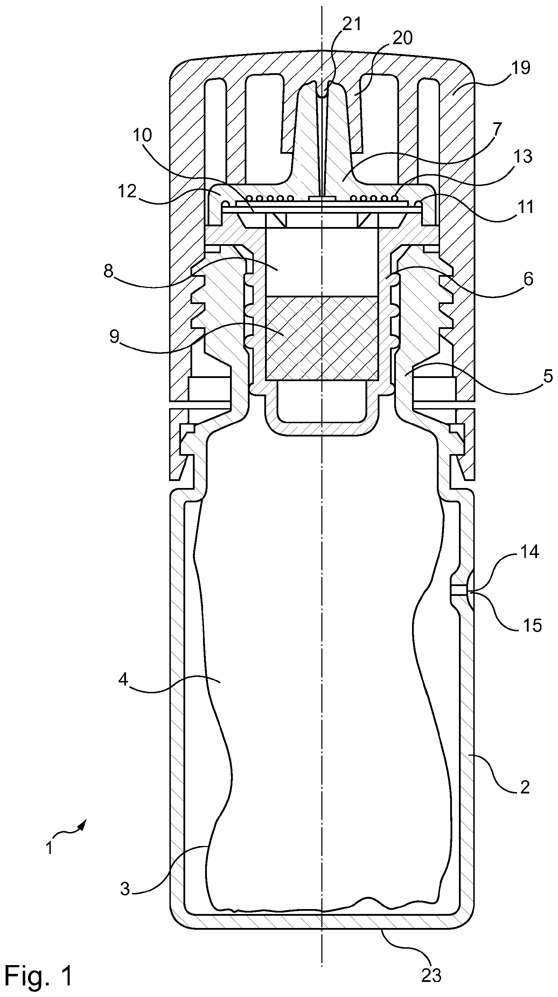

La figure 1 représente un flacon selon un premier mode de réalisation de l'invention. Le flacon comporte un réservoir 1 adapté à contenir un produit liquide, en particulier un produit liquide stérile. Le réservoir 1 comporte une paroi 2 déformable, et qui forme un volume interne du réservoir 1. La paroi 2 est en particulier configurée pour pouvoir être déformée sous une pression exercée par la main d'un utilisateur lorsque celui-ci souhaite procéder à la délivrance d'une ou plusieurs gouttes de produit.

Dans l'exemple représenté, la paroi 2 est une paroi périphérique cylindrique. La paroi 2 est élastiquement déformable, c'est-à-dire qu'elle tend spontanément à reprendre sa forme initiale après qu'une pression a cessé d'être exercée sur elle.

Le réservoir comporte, dans son volume interne, une poche souple 3. La poche souple 3 peut être une poche rapportée, formée en un matériau plastique souple, en silicone, etc. Alternativement, la poche souple 3 est formée par délamination de la surface interne de la paroi 2, c'est-à-dire que la poche souple 3 se détache de la paroi 2 lors des premières délivrances de produit. La poche souple forme un volume de réception 4 dans lequel est contenu le produit liquide. La poche souple doit donc présenter des caractéristiques de perméabilité suffisante pour assurer une bonne conservation du produit liquide qu'elle contient sur une longue période, typiquement de plusieurs semaines ou plusieurs mois. Dans l'exemple représenté, la poche souple se maintient au contact de la paroi 2 du réservoir au moins à proximité d'un col 5 du réservoir.

Une tête de distribution est rapportée au flacon. Dans l'exemple de mode de réalisation ici représenté, la tête de distribution du liquide en goutte à goutte comprend un insert 6, disposé à l'intérieur du col 5 du flacon. Un embout 7 (ou buse) de distribution goutte à goutte est fixé au (ou formé par) l'insert 6. L'insert 6 est fixé rigidement et de manière étanche dans le col 5. L'insert 6 peut par exemple être inséré en force dans le col 5. L'insert 6 est dit évidé, en ce qu'il ménage un espace 8 dans lequel est reçu un tampon poreux 9, optionnel dans le cadre de la présente invention. Le tampon poreux 9 est ici de forme cylindrique épousant celle de l'espace 8. Il est réalisé en une matière hydrophobe. Il peut notamment être constitué en un feutre à trame en polyéthylène. Le tampon 9 peut par exemple présenter une porosité équivalente d'environ 100 µm. Il a pour effet de réguler le flux de liquide distribué et d'interdire le passage du produit liquide depuis le réservoir 1 vers l'embout 7 en l'absence de compression de la paroi 2 du flacon.

Le flacon comprend également une membrane microporeuse 10. La membrane microporeuse est dite stérilisante ou antibactérienne, en ce qu'elle offre une filtration suffisamment fine pour empêcher le passage de tout ou partie des bactéries, ainsi que, le cas échéant, de certains virus.

La membrane microporeuse est disposée en aval du tampon poreux 9 et en amont de l'embout 7. La membrane protège par filtration le produit liquide stérile contenu dans la poche souple 3 du réservoir 1 des contaminations extérieures, notamment par des bactéries. Elle filtre également le produit liquide contenu lors de chaque distribution. La membrane microporeuse 10 a une taille de pores inférieure à 0,45 µm et de préférence inférieure ou égale à 0,22 µm, par exemple comprise entre 0,1 µm et 0,2 µm. Elle est avantageusement formée en polyéther sulfone. La membrane microporeuse utilisée est hydrophile (sur la totalité de sa surface) de sorte qu'elle permet, sélectivement, le passage d'un liquide aqueux. Elle s'oppose par contre au passage des gaz, notamment de l'air. Cela est particulièrement le cas lorsqu'elle est imbibée de liquide.

Ainsi, la membrane 10 se laisse traverser par un liquide aqueux sous l'effet d'un différentiel de pression entre ses deux faces, en s'opposant au passage d'air. En particulier, tant que la membrane est mouillée par le liquide, elle s'oppose fortement au passage d'air malgré l'éventuelle présence d'un différentiel de pression entre les faces de la membrane.

Dans l'exemple représenté, cette membrane 10 est interposée entre l'embout 7 et l'insert 6. Plus particulièrement, la membrane 10 est supportée contre une embase 12 de l'embout de distribution 7. En forme de disque, elle est fixée sur son pourtour par soudage thermique entre une couronne périphérique 11 de cette embase et une portée coopérante présente vers une extrémité de l'insert 6.

Ici, l'embase 12 de l'embout 7 a la forme d'un disque évidé qui s'emboîte sur l'insert 6. L'embase 12 peut comporter sur sa face interne (qui est dirigée vers le volume interne du réservoir 1) des microcanaux 13 qui facilitent le drainage du liquide vers l'orifice d'expulsion.

Le flacon comporte en outre, un moyen d'entrée d'air 14. Le moyen d'entrée d'air 14 permet à l'air de traverser la paroi 2 du flacon 1, de sorte à prendre place entre ladite paroi 2 et la poche souple 3. Dans l'exemple ici représenté, le moyen d'entrée d'air 14 est formé d'un trou 15, par exemple un trou rond de quelques millimètres de diamètre. Le trou 15 peut être formé dans un léger renfoncement de la paroi 2.

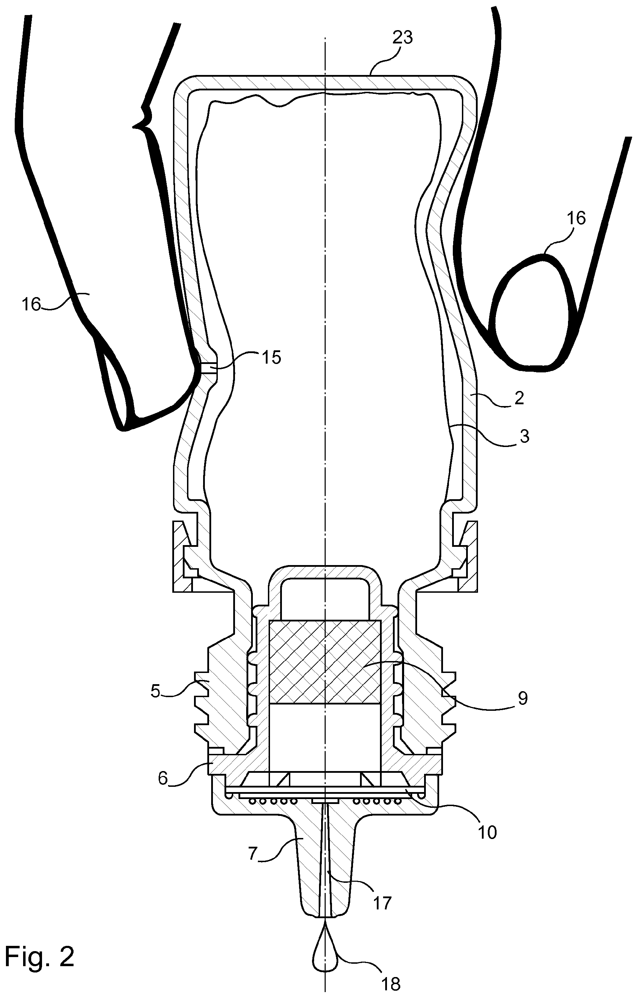

La figure 2 représente le flacon de la figure 1 lors de la délivrance du produit qu'il contient. Pour entrainer la délivrance d'une ou plusieurs gouttes de produit liquide, un utilisateur presse le flacon par exemple entre deux doigts 16, déformant ainsi la paroi 2, et réduisant son volume interne. Lors de la compression manuelle du flacon, l'un des doigts 16 est placé de sorte à obturer le trou 15, ce qui empêche l'air présent entre la paroi 2 et la poche souple 3 de s'évacuer. Il en résulte une augmentation de la pression dans le réservoir, tant pour ce qui concerne l'air présent entre la paroi 2 et la poche souple 3 que pour le produit liquide, la pression étant transmise par la poche souple 3.

La différence de pression entre le volume interne du réservoir 1 et l'air ambiant entraine un flux de produit liquide contenu dans le réservoir au travers du tampon poreux 9. Le tampon poreux 9 crée une perte de charge qui régule ce flux.

Le liquide atteint ensuite la membrane microporeuse 10, qu'il traverse librement en l'absence de mousse. Le liquide s'évacue ensuite par l'embout 7, par exemple via un canal 17 de faible section (par exemple un canal capillaire) formé dans l'embout 7 et permettant la formation de gouttes 18 régulières.

Lorsque l'utilisateur relâche la pression qu'il exerce sur le réservoir 1, son doigt 16 cesse d'obturer de manière étanche le trou 15. L'air est ainsi libre d'entrer dans l'espace formé entre la paroi 2 et la poche souple 3. Dans le même temps, la membrane hydrophile 10 s'oppose à la ré-aspiration d'air dans la poche souple via l'embout 7. Il en résulte que le volume de produit liquide expulsé sera totalement compensé par l'entrée d'un volume d'air correspondant dans le réservoir 1 entre la paroi 2 et la poche souple 3.

Le flacon permet ainsi le conditionnement de plusieurs doses et la délivrance d'un produit liquide stérile sans conservateur, en particulier d'un produit ophtalmique sans conservateur, contenant un agent tensioactif qui pourrait perturber le fonctionnement d'une membrane stérilisante (antibactérienne) bi-fonctionnelle hydrophile/hydrophobe. De nombreux autres aspects pouvant être mis en oeuvre sur un tel flacon sont illustrés aux figures suivantes.

En particulier, afin de garantir l'absence d'aspiration d'air dans la poche souple 3 et plus généralement au contact du produit liquide en amont de la membrane hydrophile 10, divers moyens peuvent être employés en alternatives ou en compléments les uns des autres. En premier lieu, un capuchon 19 amovible d'obturation de l'embout 7 peut être utilisé. Le capuchon 19 peut être en particulier adapté à se visser autour du col 5 du flacon. Le capuchon assure une étanchéité à l'air grâce à son mode de fixation sur le col 5 du flacon, et/ou grâce à des moyens complémentaires. Notamment, un cylindre de réception 20 de l'embout 7 peut être formé dans le capuchon 19, ainsi qu'un pion 21 d'obturation du canal 17. Ainsi, une fois le flacon fermé, entre deux distributions de produit, aucune entrée d'air via l'embout 7 de distribution ne peut se produire.

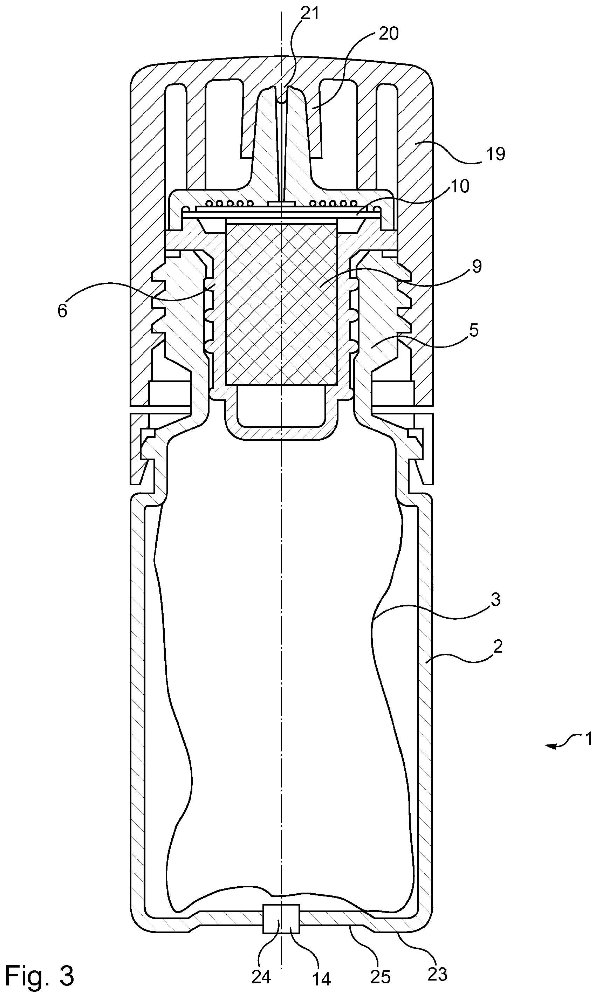

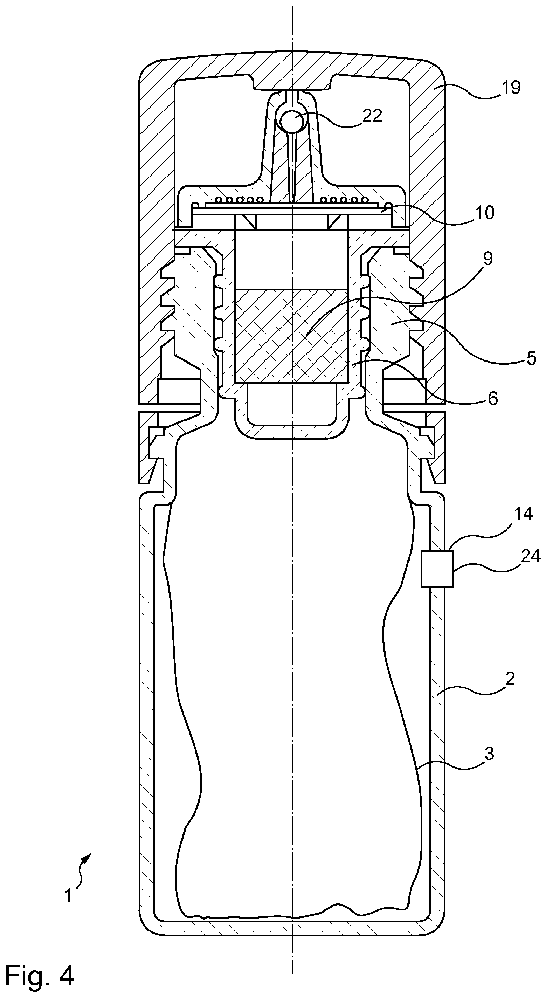

Dans le mode de réalisation de la figure 3, le tampon poreux 9 est en outre dimensionné pour être au contact ou à proximité de la membrane 10. Le tampon constitue ainsi, entre deux distributions de produit, une réserve de liquide qui perdure à mouiller la membrane, renforçant ainsi ses propriétés de sélectivité, à savoir qu'elle s'oppose au passage des gaz tout en permettant le passage des liquides. Cela limite fortement la possibilité pour l'air de pénétrer sous la membrane, en amont de celle-ci. La figure 4 présente un autre mode de réalisation de l'invention, dans lequel, en plus de l'emploi d'un capuchon 19, on s'assure que la membrane reste mouillée entre deux distributions de produit en conservant une très faible quantité de liquide en aval de la membrane. Cela peut être réalisé en dotant le flacon d'un clapet 22 dans l'embout 7, par exemple à proximité immédiate de son extrémité. Le clapet représenté à la figure 4 est un clapet à bille, mais toute autre technologie de clapet peut bien évidemment convenir, dès lors que le clapet permet, notamment en complément de l'effet de capillarité existant dans le canal 17, de faire perdurer le mouillage de la membrane 10 par du liquide situé immédiatement en aval de celle-ci. Il est donc remarquable qu'il ne s'agit pas nécessairement de créer une parfaite étanchéité en aval de la membrane, mais de s'assurer que la membrane continue à jouer le rôle anti-retour d'air qu'elle assure par son caractère sélectivement hydrophile.

Dans l'exemple de mode de réalisation représenté aux figures 1 et 2, le moyen d'entrée d'air 14 est formé d'un trou 15 pouvant être obstrué facilement par l'utilisateur. Le positionnement du trou 15, s'il doit être naturel pour l'utilisateur, peut cependant être adapté selon le flacon. Il peut être en partie haute de la paroi 2, en son milieu (selon la direction générale d'extension du flacon), ou encore sur le fond 23 du flacon.

Comme illustré à la figure 3 et à la figure 4, le trou 15 peut être remplacé par une valve unidirectionnelle 24, laissant entrer l'air dans l'espace entre la paroi 2 et la poche souple 3 mais l'empêchant de ressortir de cet espace. La valve unidirectionnelle 24 peut être une valve à lèvres souples, à bille, etc. Elle peut être située sur la paroi 2 comme représenté à la figure 4 ou sur le fond 23 du flacon comme représenté à la figure 3. Dans le mode de réalisation de la figure 3, la valve unidirectionnelle est incluse dans un renfoncement 25 formé dans le fond 23 du flacon.

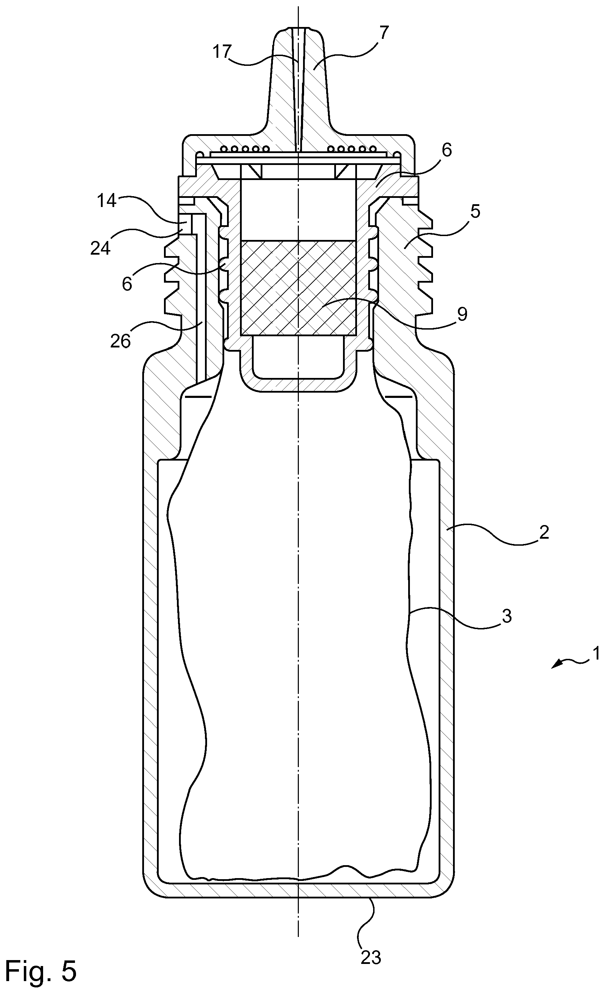

La figure 5 présente enfin un mode de réalisation dans lequel le moyen d'entrée d'air 14 est intégré en partie haute du flacon, à savoir dans l'exemple représenté dans son col 5. Un conduit 26 permet de guider l'air entrant par le moyen d'entrée d'air vers l'espace situé entre la paroi 2 et la poche souple 3. Une valve unidirectionnelle 24, formant moyen d'entrée d'air 14, est disposée à l'embouchure du conduit 26. Ce mode de réalisation permet une intégration discrète du moyen d'entrée d'air et propose un positionnement pour ce moyen qui évite tout risque d'obturation involontaire lors de la distribution du produit contenu dans le flacon.

Alternativement à une valve unidirectionnelle, le moyen d'entrée d'air 14 peut être équipé (dans tout mode de réalisation de l'invention) d'un élément perméable à l'air mais adapté à générer une perte de charge importante (de sorte que la diffusion de l'air au travers de cet élément ne peut se faire que lentement). Un fin opercule perméable à l'air, par exemple en silicone, peut être utilisé pour former cet élément. Ainsi, lors de la délivrance du produit, lorsque l'utilisateur presse le réservoir 1, un différentiel de pression important est créé mais la résistance contre l'expulsion du produit par l'embout 7 est moindre que la résistance au passage de l'air générée par l'opercule, et le temps nécessaire à la distribution est trop court pour permettre un passage significatif de l'air au travers de l'opercule.

Lorsque la pression sur le réservoir est relâchée, l'entrée d'air par l'embout 7 étant empêchée par la membrane hydrophile 10, la différence de pression entre le volume intérieur du réservoir et l'extérieur entraine le passage progressif d'air au travers de l'opercule, jusqu'à ce que les pressions soient équilibrées. La compensation du volume de produit distribué par de l'air entre la paroi 2 et la poche souple 3 est ainsi réalisée.

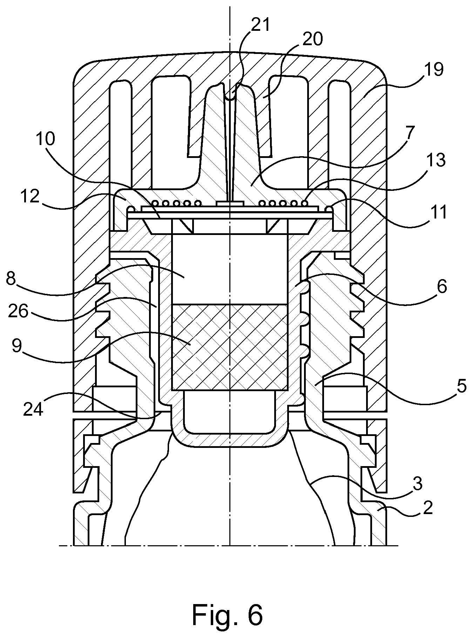

La figure 6 présente un autre mode de réalisation de l'invention, dans lequel la poche souple 3 est une poche souple rapportée et le moyen d'entrée d'air comporte un élément formant valve unidirectionnelle 24 au niveau de l'interface entre la poche souple et l'élément du flacon auquel la poche souple est liée.

Dans l'exemple ici représenté la poche souple 3 est liée à l'insert 6 (cette configuration étant d'ailleurs applicable à tout mode de réalisation comportant une poche rapportée). L'élément formant valve unidirectionnelle 24 est ouvert et permet le passage de l'air en l'absence de différence de pression entre l'extérieur du flacon et le volume interne du réservoir, ou est configuré pour s'ouvrir dès qu'une faible dépression est générée dans le volume interne du réservoir. Par contre, dès que le volume interne est en surpression comparativement à la pression extérieure au flacon (pression atmosphérique), l'élément formant valve unidirectionnelle 24 se ferme et empêche l'air de s'échapper de l'espace situé entre la paroi 2 et la poche souple 3.

En particulier, l'élément formant valve unidirectionnelle 24 peut être formé d'une fine collerette ou d'un joint plat pouvant soit être plaqué sur la sortie d'un conduit 26 reliant l'extérieur du flacon et le volume interne du réservoir, ce qui obture le conduit 26, soit au contraire se décoller ou se déformer pour libérer le conduit 26, selon le différentiel de pression entre l'intérieur et l'extérieur du flacon. Un opercule perméable à l'air tel que décrit ci-dessus peut aussi être utilisé dans une variante de ce mode de réalisation.

Le mode de réalisation de la figure 6 offre les avantages de celui de la figure 5, et est simple à mettre en oeuvre.

Dans tous les modes de réalisation présentés ci-dessous, et plus généralement dans tous les modes de réalisation de l'invention, il peut exister un intérêt à conférer un effet antibactérien aux surfaces du flacon situées en aval de la membrane 10 et susceptible d'être mouillées par le produit liquide contenu dans le flacon. Cela peut être obtenu par un traitement de ces surfaces ou un traitement du matériau constitutif de ces surfaces. Un tel traitement, à base d'ions argents ou d'ions zinc par exemple, permet d'éviter toute prolifération bactérienne sur ces surfaces. Si une faible quantité de liquide est retenue en aval de la membrane 10, le traitement évite également toute prolifération bactérienne dans cette faible quantité de liquide.

Le traitement antibactérien concerne ainsi, dans les exemples représentés, les surfaces internes de l'embout 7, notamment le canal 17, et le cas échéant le clapet 22. Avantageusement, la surface aval de la membrane 10 est également traitée pour éviter tout développement bactérien.

Bien évidemment, les différents aspects présentés ci-dessus en référence aux figures données à titre d'exemple peuvent être combinés pour former d'autres modes de réalisation de l'invention.

Le flacon ainsi développé permet le conditionnement « multidoses » d'un produit liquide stérile sans conservateur. En outre, l'absence de partie hydrophobe sur la membrane hydrophile permet le conditionnement « multidoses » d'un produit liquide stérile sans conservateur contenant un agent tensioactif. En outre, le flacon tire parti du caractère sélectif de la membrane hydrophile vis-à-vis des liquides (qu'elle laisse passer) et des gaz (au passage desquels elle s'oppose) pour empêcher l'aspiration d'air dans le volume de réception du produit. En outre, le volume de la poche s'adaptant à celui du produit liquide, tout le produit peut être distribué, sans difficulté, y compris les dernières doses présentes dans le flacon, sans déformation résiduelle de la paroi du réservoir.

## Revendications

1. Flacon pour la distribution goutte à goutte d'un produit liquide stérile contenant au moins un agent tensioactif, comportant
un réservoir (1) comportant une paroi (2) définissant un volume interne et adaptée à se déformer sous l'effet d'une pression exercée sur le réservoir (1) par un utilisateur du flacon et à reprendre spontanément sa forme d'origine après relâchement de ladite pression ;
le réservoir (1) comportant en outre une poche souple (3) dans le volume interne défini par la paroi (2) et adaptée à contenir le produit liquide stérile ;
un moyen d'entrée d'air (14) entre la paroi (2) du réservoir (1) et la poche souple (3) ; une tête rapportée sur un col (5) du réservoir (1) et comportant un embout (7) goutte à goutte comportant un orifice de distribution,
le flacon comportant en outre une membrane (10) microporeuse stérilisante hydrophile, disposée de sorte à être traversée par le liquide issu de la poche souple (3) en vue de la délivrance dudit liquide par l'embout (7) goutte à goutte,
le flacon étant **caractérisé en ce qu'**il comporte un dispositif configuré pour maintenir la membrane (10) mouillée par du produit liquide issu du réservoir (1) entre deux distributions de produit liquide,
ladite membrane (10) microporeuse étant sélectivement perméable au liquide, c'est à dire qu'elle permet le passage d'un liquide aqueux tandis qu'elle s'oppose au passage des gaz lorsqu'elle est mouillée, de sorte que la membrane s'oppose au retour d'air dans la poche souple (3) du réservoir (1) lorsque le flacon reprend sa forme d'origine après une distribution du produit liquide stérile, le volume de liquide distribué étant compensé par l'entrée d'un volume d'air correspondant entre la paroi (2) du réservoir (1) et la poche souple (3) via le moyen d'entrée d'air (14), le dispositif configuré pour maintenir la membrane (10) mouillée entre deux instillations comportant un clapet (22) positionné entre la membrane (10) et l'orifice de distribution de l'embout goutte à goutte.

2. Flacon selon la revendication 1, dans lequel la poche souple (3) est formée par délamination d'une couche interne du réservoir (1).

3. Flacon selon la revendication 1, dans lequel la poche souple (3) est une poche souple rétractable.

4. Flacon selon l'une des revendications 1 à 3, dans lequel le moyen d'entrée d'air (14) est formé d'un trou (15) dans la paroi (2) du réservoir (1) adapté à être obturé par un doigt (16) de l'utilisateur.

5. Flacon selon l'une des revendications 1 à 3, dans lequel le moyen d'entrée d'air (14) comporte un clapet unidirectionnel (24), configuré pour permettre l'entrée d'air entre la paroi (2) du réservoir (1) et la poche souple (3) et pour empêcher la sortie vers l'extérieur du réservoir (1) de l'air présent entre la paroi (2) du réservoir (1) et la poche souple (3).

6. Flacon selon l'une des revendications précédentes, dans lequel le dispositif configuré pour maintenir la membrane (10) mouillée entre deux instillations comporte un capuchon (19) amovible, rapporté sur la tête de distribution.

7. Flacon selon l'une des revendications précédentes, dans lequel le flacon comporte un tampon poreux situé entre le réservoir et la membrane microporeuse et disposé de sorte à être traversé par le liquide issu de la poche souple en vue de la délivrance dudit liquide par l'embout goutte à goutte.

8. Flacon selon l'une des revendications précédentes, dans lequel la membrane microporeuse a une taille de pores inférieure à 0,45 µm et de préférence inférieure ou égale à 0,22 µm.

9. Flacon selon l'une des revendications précédentes, dans lequel les surfaces du flacon situées entre la membrane microporeuse et l'orifice de distribution portent un revêtement bactéricide, par exemple à base d'ions argent ou d'ion zinc.

10. Flacon selon l'une des revendications précédentes, dans lequel la membrane (10) microporeuse comporte un agent bactéricide, par exemple à base d'ions argent ou d'ion zinc.

11. Flacon selon l'une des revendications précédentes, ledit flacon comportant dans son réservoir (1) un produit ophtalmique liquide stérile sans conservateur et contenant au moins un agent tensioactif.

12. Flacon selon la revendication 11, dans lequel le produit ophtalmique liquide stérile comporte entre 0,1% et 5% en volume d'agent tensioactif.

13. Flacon selon la revendication 11 ou la revendication 12, dans lequel l'au moins un agent tensioactif comporte un ou plusieurs des agents suivants:
Polyéthylène glycol 400 (PEG 400) ;
Monooléate de polyoxyéthylène sorbitane (polysorbate 80) ;
Monolaurate de polyoxyéthylène sorbitane (polysorbate 20) ;
Oxyde d'éthylène, oxyde de propylène ;
Polymère de méthyloxirane ;
Huile de ricin PEG-35 ;
Huile de ricin hydrogénée PEG-40 ;
éther cétostéarylique de macrogol ;
Polyoxypropyle glycol 407 ;
Vitamine E (polyéthylène glycol succinate) ;
Hydroxystéarate de macrogol 15 ;
Caprylocaproyl macrogol-8 glycérides ;
2-(2-éthoxyéthoxy) éthanol ;
Polyvinylpyrrolidone.

## Patentansprüche

1. Flasche zur Tropfenabgabe eines sterilen flüssigen Produkts, das mindestens ein Tensid enthält, die Folgendes aufweist:
einen Behälter (1), der eine Wand (2) aufweist, die ein Innenvolumen definiert und dazu ausgelegt ist, sich unter der Wirkung eines Drucks, der von einem Benutzer der Flasche auf den Behälter (1) ausgeübt wird, zu verformen und nach dem Loslassen des Drucks spontan seine ursprüngliche Form wieder einzunehmen;
den Behälter (1), der ferner einen weichen Beutel (3) aufweist, dessen von der Wand (2) definierte Innenvolumen dazu ausgelegt ist, das sterile flüssige Produkt aufzunehmen;
ein Lufteinlassmittel (14) zwischen der Wand (2) des Behälters (1) und dem weichen Beutel (3);
einen Kopf, der auf einen Hals (5) des Behälters (1) aufgesetzt ist und der einen Tropfenstutzen (7) mit einer Dosieröffnung aufweist,
die Flasche ferner eine mikroporöse hydrophile sterilisierende Membran (10) aufweist, die so angeordnet ist, dass sie von der aus dem weichen Beutel (3) stammenden Flüssigkeit durchdrungen wird, um die Flüssigkeit durch den Tropfenstutzen (7) abzugeben,
wobei die Flasche **dadurch gekennzeichnet ist, dass** sie eine Vorrichtung aufweist, die so eingerichtet ist, dass sie die Membran (10) zwischen zwei Flüssigproduktabgaben mit Flüssigprodukt aus dem Behälter (1) benetzt hält,
wobei die mikroporöse Membran (10) selektiv flüssigkeitsdurchlässig ist, d. h., sie ermöglicht den Durchtritt einer wässrigen Flüssigkeit, während sie dem Durchtritt von Gasen entgegensteht, wenn sie nass ist, so dass die Membran dem Rückfluss von Luft in den weichen Beutel (3) des Behälters (1) entgegensteht, wenn die Flasche nach dem Abgeben des sterilen flüssigen Produkts ihre ursprüngliche Form annimmt, wobei das abgegebene Flüssigkeitsvolumen durch das Eintreten eines entsprechenden Luftvolumens zwischen der Wand (2) des Behälters (1) und des weichen Beutels (3) über das Lufteinlassmittel (14) ausgeglichen wird, wobei die Vorrichtung so eingerichtet ist, dass sie die Membran (10) zwischen zwei Instillationen feucht hält, wobei ein Ventil (22) zwischen der Membran (10) und der Dosieröffnung des Tropfenstutzens positioniert ist.

2. Flasche nach Anspruch 1, wobei der weiche Beutel (3) durch Delaminieren einer Innenschicht des Behälters (1) gebildet wird.

3. Flasche nach Anspruch 1, wobei der weiche Beutel (3) ein schrumpfbarer weicher Beutel ist.

4. Flasche nach einem der Ansprüche 1 bis 3, wobei das Lufteinlassmittel (14) durch ein Loch (15) in der Wand (2) des Behälters (1) gebildet ist, das mit einem Finger (16) des Benutzers verschlossen werden kann.

5. Flasche nach einem der Ansprüche 1 bis 3, wobei das Lufteinlassmittel (14) ein Einwegventil (24) aufweist, das so eingerichtet ist, dass es das Eintreten von Luft zwischen der Wand (2) des Behälters (1) und dem weichen Beutel (3) ermöglicht und das Austreten der zwischen der Wand (2) des Behälters (1) und dem weichen Beutel (3) vorhandenen Luft nach außen aus dem Behälter (1) verhindert.

6. Flasche nach einem der vorhergehenden Ansprüche, wobei die Vorrichtung, die so eingerichtet ist, dass sie die Membran (10) zwischen zwei Instillationen feucht hält, eine abnehmbare Kappe (19) aufweist, die auf den Dosierkopf angebracht ist.

7. Flasche nach einem der vorhergehenden Ansprüche, wobei die Flasche einen porösen Puffer aufweist, der sich zwischen dem Behälter und der mikroporösen Membran befindet und so angeordnet ist, dass er von der aus dem weichen Beutel stammenden Flüssigkeit durchdrungen wird, um die Flüssigkeit durch den Tropfenstutzen abzugeben.

8. Flasche nach einem der vorhergehenden Ansprüche, wobei die mikroporöse Membran eine Porengröße von weniger als 0,45 µm und vorzugsweise weniger als oder gleich 0,22 µm aufweist.

9. Flasche nach einem der vorhergehenden Ansprüche, wobei die Oberflächen der Flasche, die sich zwischen der mikroporösen Membran und der Dosieröffnung befinden, eine bakterizide Beschichtung aufweisen, beispielsweise auf Basis von Silberionen oder Zinkionen.

10. Flasche nach einem der vorhergehenden Ansprüche, wobei die mikroporöse Membran (10) ein bakterizides Mittel aufweist, beispielsweise auf Basis von Silberionen oder Zinkionen.

11. Flasche nach einem der vorhergehenden Ansprüche, wobei die Flasche in ihrem Behälter (1) ein steriles, konservierungsmittelfreies, flüssiges ophthalmologisches Produkt aufweist und mindestens ein Tensid enthält.

12. Flasche nach Anspruch 11, wobei das sterile flüssige ophthalmologische Produkt zwischen 0,1 % und 5 % Volumen an Tensid enthält.

13. Flasche nach Anspruch 11 oder Anspruch 12, wobei das mindestens eine Tensid eines oder mehrere der folgenden Mittel aufweist:
Polyethylenglykol 400 (PEG 400);
Polyoxyethylensorbitan-Monooleat (Polysorbat 80);
Polyoxyethylensorbitan-Monolaurat (Polysorbat 20);
Ethylenoxid, Propylenoxid;
Methyloxiranpolymer;
Rizinusöl PEG-35;
Hydriertes Rizinusöl PEG-40;
Macrogolketostearylether;
Polyoxypropylenglykol 407;
Vitamin E (Polyethylenglykolsuccinat);
Macrogol-Hydroxystearat 15;
Caprylocaproylmacrogol-8-Glyzeride;
2-(2-Ethoxyethoxy)ethanol;
Polyvinylpyrrolidon.

## Claims

1. Bottle for dripwise dispensing a sterile liquid product containing at least one surfactant, comprising
a reservoir (1) comprising a wall (2) defining an internal volume and adapted to deform under the effect of a pressure exerted on the reservoir (1) by a user of the bottle and to spontaneously resume its original shape after releasing said pressure;
the reservoir (1) further comprising a flexible pouch (3) in the internal volume defined by the wall (2) and adapted to contain the sterile liquid product;
an air inlet means (14) between the wall (2) of the reservoir (1) and the flexible pouch (3);
a head attached to a neck (5) of the reservoir (1) and comprising a drip tip (7) comprising a dispensing orifice,
the bottle further comprising a microporous hydrophilic sterilising membrane (10), arranged so as to be passed through by the liquid from the flexible pouch (3) with a view to delivering said liquid by the drip tip (7),
the bottle being **characterised in that** it includes a device configured to keep the membrane (10) wetted by liquid product from the reservoir (1) between two liquid-product dispensings,
said microporous membrane (10) being selectively permeable to the liquid, i.e. allowing the passage of an aqueous liquid while opposing the passage of gases when it is wetted, so that the membrane opposes the return of air into the flexible pouch (3) of the reservoir (1) when the bottle resumes its original shape after a dispensing of the sterile liquid product, the volume of liquid dispensed being compensated for by the entry of a corresponding volume of air between the wall (2) of the reservoir (1) and the flexible pouch (3) via the air inlet means (14), the device configured to keep the membrane (10) wetted between two instillations comprising a valve (22) positioned between the membrane (10) and the dispensing orifice of the drip tip.

2. Bottle according to claim 1, wherein the flexible pouch (3) is formed by delamination of an inner layer of the reservoir (1).

3. Bottle according to claim 1, wherein the flexible pouch (3) is a retractable flexible pouch.

4. Bottle according to one of claims 1 to 3, wherein the air inlet means (14) is formed by a hole (15) in the wall (2) of the reservoir (1) adapted to be closed by a finger (16) of the user.

5. Bottle according to one of claims 1 to 3, wherein the air inlet means (14) includes a one-way valve (24), configured to allow air to enter between the wall (2) of the reservoir (1) and the flexible pouch (3) and to prevent the air present between the wall (2) of the reservoir (1) and the flexible pouch (3) exiting to the outside of the reservoir (1).

6. Bottle according to one of the preceding claims, wherein the device configured to keep the membrane (10) wetted between two instillations includes a removable cap (19), attached to the dispensing head.

7. Bottle according to one of the preceding claims, wherein the bottle includes a porous pad located between the reservoir and the microporous membrane and arranged so as to have the liquid from the flexible pouch passing through with a view to delivering said liquid by the drip tip.

8. Bottle according to one of the preceding claims, wherein the microporous membrane has a pore size of less than 0.45 µm and preferably less than or equal to 0.22 µm.

9. Bottle according to one of the preceding claims, wherein the surfaces of the bottle located between the microporous membrane and the dispensing orifice carry a bactericidal coating, for example based on silver ions or zinc ion.

10. Bottle according to one of the preceding claims, wherein the microporous membrane (10) includes a bactericidal agent, for example based on silver ions or zinc ion.

11. Bottle according to one of the preceding claims, said bottle comprising in its reservoir (1) a preservative-free sterile liquid ophthalmic product containing at least one surfactant.

12. Bottle according to claim 11, wherein the sterile liquid ophthalmic product comprises between 0.1% and 5% surfactant by volume.

13. Bottle according to claim 11 or claim 12, wherein the at least one surfactant includes one or more of the following agents:
Polyethylene glycol 400 (PEG 400);
Polyoxyethylene sorbitan monooleate (polysorbate 80);
Polyoxyethylene sorbitan monolaurate (polysorbate 20);
Ethylene oxide, propylene oxide;
Methyloxirane polymer;
PEG-35 castor oil;
PEG -40 hydrogenated castor oil
Macrogol ketostearyl ether;
Polyoxypropylene glycol 407;
Vitamin E (polyethylene glycol succinate);
Macrogol hydroxystearate 15;
Caprylocaproyl macrogol-8 glycerides;
2-(2-ethoxyethoxy) ethanol;
Polyvinylpyrrolidone.
